# EUROPÄISCHE PATENTSCHRIFT

(11) **EP 2 337 509 B1**
(45) Veröffentlichungstag und Bekanntmachung des Hinweises auf die Patenterteilung: **17.07.2013**
(21) Anmeldenummer: 09744052.3
(22) Anmeldetag: 02.10.2009
(51) Int. Cl.: A61B 17/34, A61M 39/22

(54) **VORRICHTUNG ZUR UMKEHR DES BLUTFLUSSES FÜR EINE EXTRAKORPORALE BLUTBEHANDLUNGSVORRICHTUNG UND VERFAHREN ZUR FESTSTELLUNG DER UMKEHR DES BLUTFLUSSES BEI EINER EXTRAKORPORALEN BLUTBEHANDLUNG**
DEVICE FOR REVERSING THE BLOOD FLOW FOR AN EXTRACORPOREAL BLOOD TREATMENT DEVICE AND METHOD FOR DETERMINING THE REVERSAL OF THE BLOOD FLOW DURING AN EXTRACORPOREAL BLOOD TREATMENT
DISPOSITIF POUR INVERSER LE FLUX SANGUIN POUR UN DISPOSITIF DE TRAITEMENT EXTRACORPOREL DU SANG ET PROCÉDÉ POUR DÉTERMINER L'INVERSION DU FLUX SANGUIN LORS D'UN TRAITEMENT EXTRACORPOREL DU SANG

(30) Priorität: 08.10.2008 DE 102008050849
(43) Veröffentlichungstag der Anmeldung: 29.06.2011
(73) Patentinhaber: Fresenius Medical Care Deutschland GmbH, 61352 Bad Homburg v.d.H. (DE)
(72) Erfinder: MAIERHOFER, Andreas, 97422 Schweinfurt (DE); GROSS, Malte, 89081 Ulm (DE)
(74) Vertreter: Oppermann, Frank
(86) Internationale Anmeldenummer: PCT/EP2009/007066
(87) Internationale Veröffentlichungsnummer: WO 2010/040478

(56) Entgegenhaltungen:
- WO-A1-2007/041843
- WO-A2-2006/042016
- JP-A- 2002 333 079
- US-A1- 2004 059 315

## Beschreibung

Die Erfindung betrifft eine Vorrichtung zur Umkehr des Blutflusses für eine extrakorporale Blutbehandlungsvorrichtung, die einen extrakorporalen Blutkreislauf umfasst, der eine von einem Patienten abgehende zu einer Blutbehandlungseinheit führende arterielle Blutleitung und eine von der Blutbehandlungseinheit abgehende zu dem Patienten führende venöse Blutleitung aufweist. Darüber hinaus betrifft die Erfindung eine extrakorporale Blutbehandlungsvorrichtung mit einer Vorrichtung zur Umkehr des Blutflusses sowie ein Verfahren zur Feststellung der Umkehr des Blutflusses bei einer extrakorporalen Blutbehandlung.

Bei einer extrakorporalen Blutbehandlungsvorrichtung, beispielsweise einer Hämodialysevorrichtung, durchströmt das zu behandelnde Blut in einem extrakorporalen Blutkreislauf die Blutkammer eines durch eine semipermeable Membran in die Blutkammer und eine Dialysierflüssigkeitskammer unterteilten Dialysators, während in einem Dialysierflüssigkeitssystem Dialysierflüssigkeit die Dialysierflüssigkeitskammer des Dialysators durchströmt. Der extrakorporale Blutkreislauf weist eine arterielle Schlauchleitung auf, die zu der Blutkammer führt, und eine venöse Schlauchleitung auf, die von der Blutkammer abgeht. Die Schlauchleitungen der extrakorporalen Blutbehandlungsvorrichtung werden im Allgemeinen als zur einmaligen Verwendung bestimmtes Disposable bereitgestellt. Die bekannten Blutbehandlungsvorrichtungen verfügen über eine Blutpumpe, die in der Regel stromauf der Blutkammer des Dialysators angeordnet ist, um einen ausreichenden Blutfluss im extrakorporalen Blutkreislauf sicherzustellen.

Für hocheffiziente Hämodialysebehandlungen ist es notwendig, das zu reinigende Blut des Patienten dem extrakorporalen Blutkreislauf mit hinreichend hohen Förderraten zuzuführen. Hierzu wird bevorzugt eine operativ hergestellte Verbindung zwischen Arterie und Vene verwendet. Durch die Umgehung des einen hohen Flusswiderstand darstellenden peripheren Gefäßsystems werden in der Fistel oder dem Shunt relativ hohe Blutflüsse erreicht, die im Folgenden als Access-Flüsse bezeichnet werden. Der Anschluss des extrakorporalen Blutkreislaufs erfolgt bevorzugt mittels zweier Kanülen, wobei über die der Arterie zugewandte Kanüle das Blut entnommen und über die der Vene zugewandte Kanüle das Blut nach der Passage durch den extrakorporalen Blutkreislauf wieder zurückgegeben wird.

Im Laufe der Zeit können sich aus unterschiedlichen Gründen in Fistel oder Shunt Stenosen bilden, die zum Absinken des Access-Flusses führen. Ist der Access-Fluss geringer als der Fluss im extrakorporalen Kreislauf, so führt dies zum Absinken der Reinigungsleistung, da sich nun bereits gereinigtes (venöses) Blut mit ungereinigtem (arteriellem) Blut vermischt. Dieser Vorgang wird als Access-Rezirkulation bezeichnet. Neben dem Absinken des Access-Flusses kann dieser Vorgang auch durch eine ungünstige Positionierung von arterieller und venöser Kanüle zueinander begünstigt werden.

Durch die regelmäßige Messung des Access-Flusses sollen frühzeitig sich bildende Stenosen erkannt werden, um bei einem Absinken des Flusses unter einen kritischen Wert frühzeitig Maßnahmen zur Wiederherstellung des Access-Flusses ergreifen zu können.

Eine Messung des Access-Flusses ist beispielsweise durch die Duplex-Sonographie möglich. Diese erfordert jedoch einen ausgebildeten Arzt sowie ein kostenintensives Diagnosegerät. Zudem ist dieses Verfahren bei stark adipösen Patienten nicht einsetzbar. Als Standardverfahren zur Bestimmung des Access-Flusses hat sich die Krivitski-Methode (Kidney Int (Jul) 48:244-250 1995) etabliert. Hierbei wird bei vertauschter venöser und arterieller Nadel ein Bolus Kochsalzlösung in das extrakorporale Blutschlauchsystem injiziert und mittels Ultraschallsensoren am arteriellen und venösen Blutschlauch detektiert. Diese Methode erfordert ein kostenintensives Zusatzgerät sowie die manuelle Gabe von Kochsalz.

Aus der EP 0 928 614 B1 ist ein Verfahren zur Bestimmung des Access-Flusses bekannt, das außer dem Dialysegerät keine weiteren Messgeräte benötigt und ohne die manuelle Injektion einer Indikatorlösung auskommt. Hierbei wird nacheinander bei konstanten Blut- und Dialysatflüssen die Clearance bei normaler und bei inverser Anordnung der Kanülen bestimmt und der Access-Fluss aus den beiden Clearance-Werten berechnet.

Zur Umkehr des Blutflusses im extrakorporalen Blutkreislauf ist aus der WO 2006/042016 A2 eine Vorrichtung bekannt, die einen möglichen Blutverlust bei der Vertauschung der Kanülen verhindert, das Infektionsrisiko verringert und die Bedienung erleichtert. Weiterhin ist eine am Dialysegerät implementierte Bedienerführung zur Durchführung einer Access-Fluss-Messung unter Verwendung der bekannten Vorrichtung zur Flussumkehr bekannt.

Zur Bestimmung des Access-Flusses wird jeweils eine Messung der Clearance vor und nach der Umkehr des Blutflusses im extrakorporalen Blutkreislauf durchgeführt. Die im Dialysegerät implementierte Bedienerführung gibt dem Benutzer vor, wann die Flussumkehr durchzuführen ist. Nachteilig ist jedoch, dass nicht automatisch erkannt wird, ob die Flussumkehr tatsächlich stattgefunden hat. Wenn fälschlicherweise keine Flussumkehr erfolgt ist, so kann die Messung zu einem zu hohen Wert für den Access-Fluss führen. Dies kann zur Folge haben, dass Probleme am Gefäßzugang nicht erkannt werden, zumal der Benutzer annimmt, eine korrekte Messung durchgeführt zu haben.

Des Weiteren besteht die Gefahr, dass nach einer Messung mit umgekehrtem Blutfluss die Umkehrung des Blutflusses in die ursprüngliche Richtung nicht erfolgt. Dies führt dann wegen der bei Flussumkehr erhöhten Access-Rezirkulation zu einer verringerten Dialysedosis.

Für eine automatische Bestimmung der Dialysedosis durch das Dialysegerät ist die Kenntnis der aktuellen Clearance zu jedem Zeitpunkt der Behandlung erforderlich. Hierzu werden zu verschiedenen Zeitpunkten Messungen durchgeführt. Erfolgt zwischen den Messungen eine Änderung der die Clearance beeinflussenden Parameter, insbesondere des Blut- und Dialysatflusses, so ist auf der Grundlage der erfolgten Messungen und der bekannten Behandlungsparameter eine Interpolation möglich (DE 19928407). Da die Flussumkehr die Clearance beeinflusst, ist für die Bestimmung der Dialysedosis die genaue Kenntnis des Zeitraums der Flussumkehr und der Dauer der Behandlung mit normaler Flussrichtung nötig.

Zur Identifizierung und Lokalisierung von Gegenständen findet eine unter dem englischen Begriff Radio Frequency Identification (RFID) bekannte Technik allgemein Verwendung. Die Identifizierung der Gegenstände erfolgt mit Hilfe von elektromagnetischen Wellen. Ein RFID-System besteht aus einem am Gegenstand befindlichen Transponder sowie einem Lesegerät zum Auslesen der Kennung des Transponders. Das Lesegerät erzeugt ein elektromagnetisches Hochfrequenzfeld geringer Reichweite, mit dem im Allgemeinen nicht nur die Daten übertragen werden, sondern auch der Transponder mit Energie versorgt wird.

RFID-Transponder sind in unterschiedlicher Ausbildung bekannt. Sämtliche RFID-Transponder verfügen über eine Antenne, die mit einem Transceiver verbunden ist. Neben Antenne und Transceiver sind ein permanenter Speicher sowie weitere Schaltkreise vorgesehen.

Aus der JP 22333079 A2, JP 24232671 A2 und JP 2002333079 A2 sind Ventile bekannt, die über einen RFID-Transponder verfügen, um erkennen zu können, ob das Ventil geöffnet oder geschlossen ist. Die US 2007/0277824 A1 beschreibt eine für Diagnosezwecke vorgesehene Vorrichtung, bei der die richtige Positionierung eines Einsatzstückes mittels eines RFID-Transponders überwacht wird.

Der Erfindung liegt die Aufgabe zugrunde, eine Vorrichtung zur Umkehr des Blutflusses für eine extrakorporale Blutbehandlungsvorrichtung zu schaffen, die eine Erhöhung der Sicherheit der extrakorporalen Blutbehandlung sowie eine Vereinfachung der Messung von Parametern der Blutbehandlung mit hoher Sicherheit erlaubt.

Darüber hinaus liegt der Erfindung die Aufgabe zugrunde, ein einfach zu handhabendes und sicher anzuwendendes Verfahren zur Feststellung der Umkehr des Blutflusses bei einer extrakorporalen Blutbehandlung anzugeben.

Eine weitere Aufgabe der Erfindung ist, eine Blutbehandlungsvorrichtung zu schaffen, die auf einfache Weise mit hoher Sicherheit eine Flussumkehr zur Messung von Parametern der Blutbehandlung ermöglicht.

Die Lösung dieser Aufgaben erfolgt erfindungsgemäß mit den Merkmalen der unabhängigen Patentansprüche. Vorteilhafte Ausführungsformen der Erfindung sind Gegenstand der Unteransprüche.

Die erfindungsgemäße Vorrichtung zur Umkehr des Blutflusses für eine extrakorporale Blutbehandlungsvorrichtung verfügt über zwei Ventilkörper, die gegeneinander verdrehbar sind. Der eine Ventilkörper weist einen ersten patientenseitigen Anschluss für einen ersten patientenseitigen Schlauchleitungsabschnitt der Blutleitung und einen zweiten patientenseitigen Anschluss für einen zweiten patientenseitigen Schlauchleitungsabschnitt der Blutleitung auf, während der zweite Ventilkörper einen ersten maschinenseitigen Anschluss für einen ersten maschinenseitigen Schlauchleitungsabschnitt der Blutleitung und einen zweiten maschinenseitigen Anschluss für einen zweiten maschinenseitigen Schlauchleitungsabschnitt der Blutleitung aufweist.

Die beiden Ventilkörper können zwischen einer ersten Position, die einem normalen Blutfluss entspricht, und einer zweiten Position, die einem umgekehrten Blutfluss im extrakorporalen Blutkreislauf entspricht, um eine gemeinsame Achse gegeneinander verdreht werden. In der ersten Position wird eine Flüssigkeitsverbindung zwischen dem ersten patientenseitigen Anschluss und dem ersten maschinenseitigen Anschluss einerseits und zwischen dem zweiten patientenseitigen Anschluss und dem zweiten maschinenseitigen Anschluss andererseits hergestellt, während in der zweiten Position eine Flüssigkeitsverbindung zwischen dem ersten patientenseitigen Anschluss und dem zweiten maschinenseitigen Anschluss einerseits und zwischen dem zweiten patientenseitigen Anschluss und dem ersten maschinenseitigen Anschluss andererseits hergestellt wird. Die erfindungsgemäße Vorrichtung ist zur einmaligen Verwendung bestimmt, wobei die ebenfalls zur einmaligen Verwendung bestimmten Schlauchleitungen an den maschinen- und patientenseitigen Anschlüssen angeschlossen werden können.

Die erfindungsgemäße Vorrichtung zur Umkehr des Blutflusses zeichnet sich durch eine Kommunikationseinrichtung zum Empfangen eines elektromagnetischen Hochfrequenzfeldes von einer Leseeinrichtung der extrakorporalen Blutbehandlungsvorrichtung und zum Erzeugen mindestens einer die Position der Ventilkörper kennzeichnenden Kennung aus, die von der Leseeinrichtung auslesbar ist.

Die erfindungsgemäße Kommunikationseinrichtung der Blutbehandlungsvorrichtung erlaubt eine Identifikation der Flussrichtung, ohne dass eine Verbindung zwischen der Blutbehandlungsvorrichtung und der Vorrichtung zur Umkehr des Blutflusses herzustellen ist. Die Vorrichtung zur Flussumkehr kann einfach gehandhabt werden, da die Vorrichtung lediglich in den extrakorporalen Blutkreislauf integriert zu werden braucht, ohne jedoch elektrisch an die Blutbehandlungsvorrichung angeschlossen werden zu müssen.

Eine erste alternative Ausführungsform der erfindungsgemäßen Vorrichtung sieht vor, dass die Kommunikationseinrichtung einen ersten RFID-Transponder und einen zweiten RFID-Transponder aufweist. Dabei sendet der erste RFID-Transponder eine erste Kennung, die die erste Position der Ventilkörper kennzeichnet, und der zweite RFID-Transponder sendet eine die zweite Position kennzeichnende Kennung. Es können also die bekannten RFID-Transponder verwendet werden, die auch als RFID-tag bezeichnet werden. Diese zeichnen sich durch geringe Abmessungen und geringe Kosten aus. Die bestehende Kennung des jeweiligen RFID-Transponders erlaubt eine eindeutige Identifikation der Richtung des Blutflusses.

Eine erste Variante der ersten Ausführungsform der Erfindung beruht darauf, dass nur einer der beiden RFID-Transponder aktiv ist. Dies wird durch eine elektrische Abschirmung des anderen RFID-Transponders erreicht. Bei dieser Ausführungsform ist der erste RFID-Transponder an einer die erste Position kennzeichnenden Stelle vorzugsweise an der Innenseite des ersten oder zweiten Ventilkörpers angeordnet, während der zweite RFID-Transponder an einer die zweite Position kennzeichnenden Stelle vorzugsweise an der Innenseite des ersten oder zweiten Ventilkörpers angeordnet ist. Der erste und/oder zweite Ventilkörper sind derart elektrisch abgeschirmt, dass in der ersten Position nur der erste RFID-Transponder zum Senden der die erste Position kennzeichnenden Kennung von der Leseeinrichtung auslesbar ist, während in der zweiten Position nur der zweite RFID-Transponder zum Senden der die zweite Positionen kennzeichnenden Kennung von der Leseeinrichtung auslesbar ist. Dabei ist unerheblich, ob dabei die Transponder an dem einen oder anderen Ventilkörper angeordnet sind. Entscheidend ist, dass durch Verdrehen der Ventilkörper in die beiden Positionen der eine Transponder elektrisch abgeschirmt und der andere Transponder nicht elektrisch abgeschirmt ist.

Die elektrische Abschirmung der Transponder kann durch geeignete Metallisierungen an der Innen- oder Außenseite des jeweiligen Ventilkörpers erfolgen. Es ist aber auch möglich, den Ventilkörper aus einem für das elektromagnetische Hochfrequenzfeld undurchlässigen Material herzustellen.

Bei einer besonders bevorzugten Ausführungsform sind der erste und der zweite RFID-Transponder an der Innenseite des ersten Ventilkörpers auf einer senkrecht zur Drehachse der Ventilkörper liegenden Achse einander gegenüberliegend angeordnet, wobei die Innenseite des zweiten Ventilkörpers bis auf einen Ausschnitt zur Abschirmung des RFID-Transponders metallisiert ist, der in der ersten Position dem ersten RFID-Transponder und in der zweiten Position dem zweiten RFID-Transponder gegenüberliegt. Folglich ist in der ersten Position nur der erste RFID-Transponder aktiv, während in der zweiten Position nur der zweite RFID-Transponder aktiv ist. Dadurch wird eine einfache aber sichere Identifikation der Flussrichtung erzielt.

Bei der besonders bevorzugten Ausführungsform ist nicht zwingend erforderlich, dass auch derjenige Ventilkörper zur elektrischen Abschirmung metallisiert ist, der dem Ventilkörper gegenüberliegt, der bis auf einen Ausschnitt metallisiert ist. Zur besseren Abschirmung kann aber auch dieser Ventilkörper metallisiert sein.

Eine zweite Variante der ersten Ausführungsform sieht nicht eine elektrische Abschirmung des jeweiligen RFID-Transponders vor, sondern die Abtrennung der Antenne von dem Transceiver des jeweiligen Transponders. In der ersten Position ist nur der Transceiver des ersten RFID-Transponders mit einer Antenne verbunden, während in der zweiten Position nur der Transceiver des zweiten RFID-Transponders mit einer Antenne verbunden ist. Dadurch wird erreicht, dass die Kennung nur des einen der beiden RFID-Transponder von der Leseeinrichtung zur Identifikation der Flussrichtung auslesbar ist.

Die beiden RFID-Transponder können über jeweils eine Antenne oder auch eine gemeinsame Antenne verfügen. Vorzugsweise verfügen beide RFID-Transponder nur über eine gemeinsame Antenne, die in Abhängigkeit von der Position der Ventilkörper mit dem Transceiver des jeweiligen Transponders verbunden oder von dem Transceiver getrennt ist.

Die gemeinsame Antenne und der Transceiver des jeweiligen Transponders sind über elektrische Verbindungen verbunden, die in dem Ventilkörper ausgebildet sind. Vorzugsweise ist eine dauerhafte erste elektrische Verbindung zwischen der Antenne und dem Tranceiver vorgesehen, wobei die zweite elektrische Verbindung mit einem der beiden Transceiver der Transponder in Abhängigkeit von der Position der Ventilkörper über Kontakte an den Ventilkörpern hergestellt wird.

Die RFID-Transponder sind vorzugsweise passive Transponder, die ihre Energie über das elektromagnetische Hochfrequenzsignal der Leseeinrichtung beziehen. Daher ist eine Stromversorgung für die zur einmaligen Verwendung bestimmte Vorrichtung zur Flussumkehr nicht erforderlich. Grundsätzlich können aber auch aktive RFID-Transponder eingesetzt werden.

Eine zweite alternative Ausführungsform der erfindungsgemäßen Vorrichtung zeichnet sich dadurch aus, dass zur Identifikation der Flussrichtung nur ein einziger RFID-Transponder Verwendung findet, der vorzugsweise wieder ein passiver Transponder ist.

Bei dieser Ausführungsform ist eine die erste und zweite Position der Ventilkörper detektierende Einrichtung vorgesehen, die derart mit dem RFID-Transponder zusammenwirkt, das von der Leseeinrichtung zwei unterschiedliche Kennungen auslesbar ist, von denen die eine die erste Position und die andere Kennung die zweite Position der Ventilkörper kennzeichnet.

Die erfindungsgemäße Blutbehandlungsvorrichtung zeichnet sich durch eine Leseeinrichtung aus, die derart ausgebildet ist, dass die die erste oder zweite Position kennzeichnende Kennung der Kommunikationseinrichtung auslesbar ist.

Die erfindungsgemäße Blutbehandlungsvorrichtung, die über eine Einrichtung zur Bestimmung eines Parameters der Blutbehandlung auf der Grundlage einer ersten und zweiten Messung einer charakteristischen Größe vor bzw. nach Umkehr des Blutflusses verfügt, weist vorzugsweise eine Alarmeinheit auf, die derart mit der Einrichtung zur Bestimmung eines Parameters der Blutbehandlung zusammenwirkt, dass die Alarmeinheit einen akustischen und/oder optischen Alarm gibt, wenn nach der ersten Messung und/oder nach der zweiten Messung die Leseeinrichtung nicht die jeweilige Kennung nicht empfangen hat, die die erste bzw. zweite Position kennzeichnet. Dadurch wird sichergestellt, dass tatsächlich der Blutfluss für die Messung umgekehrt worden ist und/oder nach der Messung wieder der normale Blutfluss eingestellt worden ist. Dabei ist unerheblich, welche Parameter der Blutbehandlung bestimmt werden sollen. Allein entscheidend ist, dass die Messung eine Umkehr des Blutflusses erfordert. Beispielsweise kann die Clearance gemessen werden.

In der Praxis stellt sich das Problem, dass mehrere Blutbehandlungsvorrichtungen in enger Nachbarschaft in einem Behandlungszentrum betrieben werden, so dass eine eindeutige Zuordnung zwischen der Leseeinrichtung der Blutbehandlungsvorrichtung und der zugehörigen Vorrichtung zur Flussumkehr sichergestellt sein muss. Eine bevorzugte Ausführungsform der erfindungsgemäßen Blutbehandlungsvorrichtung verfügt daher über eine Einrichtung zur Erkennung der Kommunikationseinrichtung der zugehörigen Vorrichtung zur Umkehr des Blutflusses. Die Einrichtung zur Erkennung der Kommunikationseinrichtung weist Mittel auf, die derart ausgebildet sind, dass ein Wechsel zwischen der die erste und zweite Position kennzeichnenden Kennung innerhalb eines vorgegebenen Zeitintervalls erkannt werden kann. Der Benutzer kann also durch Verdrehen der Ventilkörper der Vorrichtung zur Flussumkehr, die der Blutbehandlungsvorrichtung zugeordnet ist, ein Identifikationssignal erzeugen, das von der zugehörigen Blutbehandlungsvorrichtung erkannt wird.

Die erfindungsgemäße Vorrichtung kann nicht nur zur Umkehr des Blutflusses in einem extrakorporalen Blutkreislauf einer extrakorporalen Blutbehandlungsvorrichtung, sondern auch zur Flussumkehr in allen anderen Flüssigkeitssystemen verwendet werden. Ein Beispiel für eine Anwendung der erfindungsgemäßen Vorrichtung außerhalb eines extrakorporalen Blutkreislaufs ist die Umkehr des Flusses, um einen in einem Flüssigkeitssystem angeordneten Filter zu spülen.

Im Folgenden werden verschiedene Ausführungsbeispiele der Erfindung unter Bezugnahme auf die Zeichnungen näher erläutert.

Es zeigen:
- Fig. 1: Ein Ausführungsbeispiel der erfindungsgemäßen Vorrichtung zur Umkehr des Blutflusses im extrakorporalen Blutkreislauf in perspektivischer Darstellung,
- Fig. 2: einen Schnitt durch die Vorrichtung von Fig. 1 entlang der Linie II-II,
- Fig. 3A: ein Ausführungsbeispiel der erfindungsgemäßen extrakorporalen Blutbehandlungsvorrichtung mit der Vorrichtung zur Umkehr des Blutflusses in stark vereinfachter schematischer Darstellung, wobei der Blutfluss nicht umgekehrt ist,
- Fig. 3B: die extrakorporale Blutbehandlungsvorrichtung von Fig. 3A, wobei der Blutfluss umgekehrt ist,
- Fig. 4A: eine perspektivische Darstellung des ersten Ventilkörpers einer ersten Ausführungsform der erfindungsgemäßen Vorrichtung zur Flussumkehr,
- Fig. 4B: eine perspektivische Darstellung des zweiten Ventilkörpers der ersten Ausführungsform der erfindungsgemäßen Vorrichtung zur Flussumkehr,
- Fig. 5A: der erste Ventilkörper einer zweiten Ausführungsform der erfindungsgemäßen Vorrichtung zur Flussumkehr in perspektivischer Darstellung,
- Fig. 5B: der zweite Ventilkörper der zweiten Ausführungsform der erfindungsgemäßen Vorrichtung zur Flussumkehr in perspektivischer Darstellung,
- Fig. 6A: der erste Ventilkörper einer weiteren Ausführungsform der erfindungsgemäßen Vorrichtung zur Flussumkehr und
- Fig. 6B: der zweite Ventilkörper der weiteren Ausführungsform der erfindungsgemäßen Vorrichtung zur Flussumkehr.

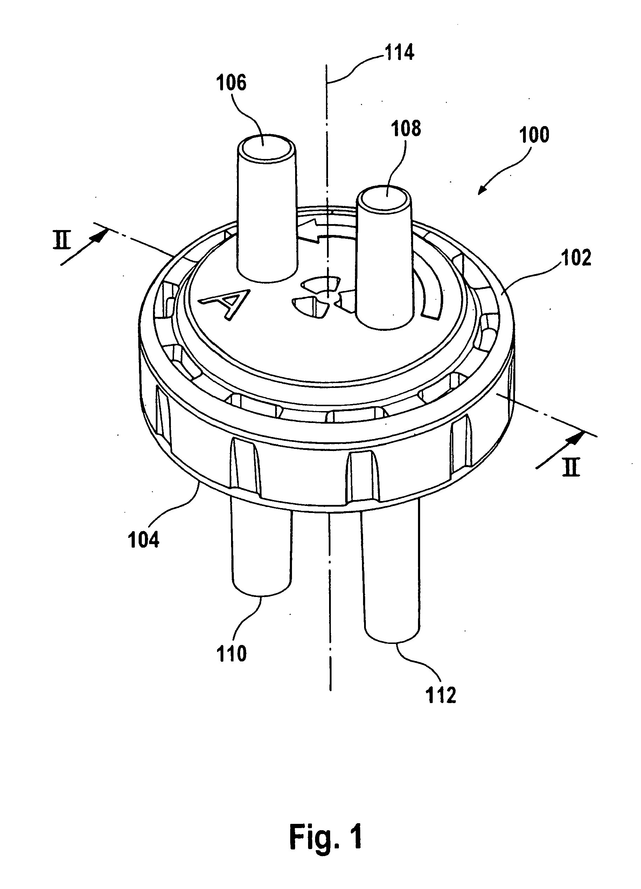

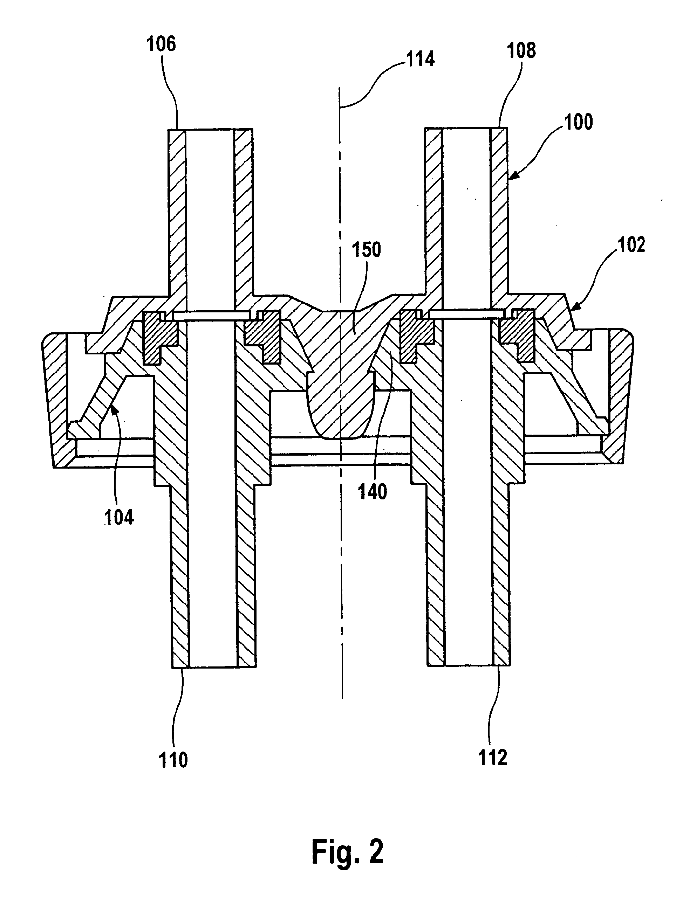

Fig. 1 zeigt in perspektivischer Darstellung die erfindungsgemäße Vorrichtung zur Flussumkehr, die zur einmaligen Verwendung bestimmt ist. Die Vorrichtung 100 zur Flussumkehr besteht aus einem ersten in Fig. 1 unteren Ventilkörper 104 und einem zweiten in Fig. 1 oberen Ventilkörper 102. Fig. 2 zeigt die Vorrichtung 100 zur Flussumkehr in geschnittener Darstellung.

Die Vorrichtung 100 zur Flussumkehr gehört als solche zum Stand der Technik. Die erfindungsgemäße Vorrichtung zur Flussumkehr unterscheidet sich von der bekannten Vorrichtung dadurch, dass die erfindungsgemäße Vorrichtung über eine Kommunikationseinrichtung verfügt, die nachfolgend noch im Einzelnen beschrieben wird. Die Figuren 1 und 2 zeigen die bekannte Vorrichtung zur Flussumkehr, wobei die Kommunikationseinrichtung nicht dargstellt ist.

Der Aufbau und die Funktionsweise der bekannten Vorrichtung zur Flussumkehr (Fig. 1 und 2) ist in der WO 2006/042016 A2 im Einzelnen beschrieben, auf die zum Zwecke der Offenbarung ausdrücklich Bezug genommen wird. Da die Vorrichtung zur Flussumkehr in der WO 2006/042016 A2 bereits im Einzelnen beschrieben ist, werden nachfolgend nur die für die Erfindung wesentlichen Komponenten erläutert.

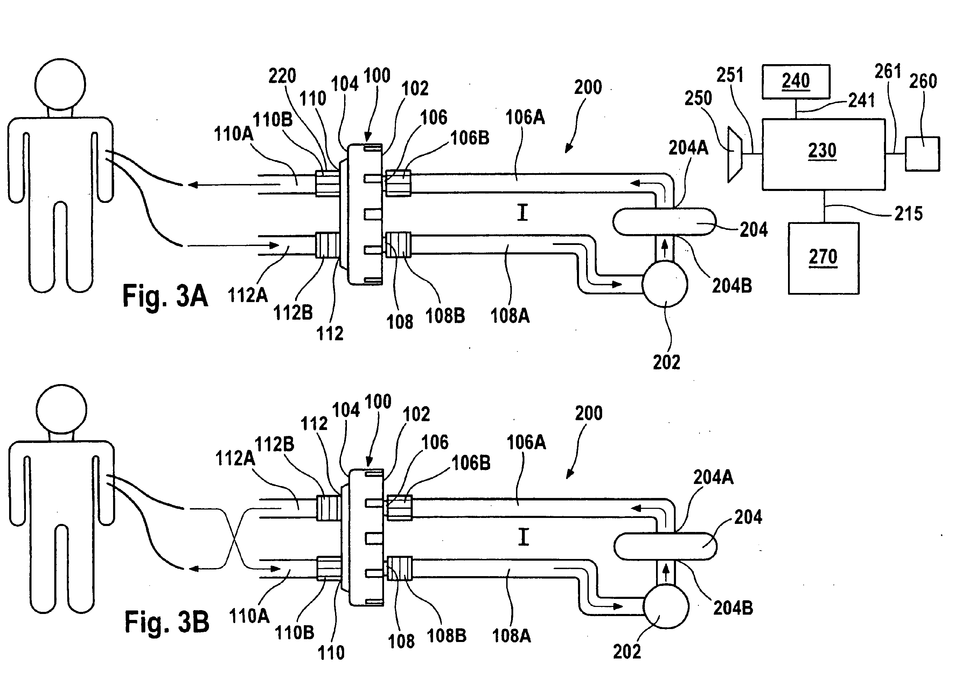

Die Vorrichtung zur Flussumkehr wird in den extrakorporalen Blutkreislauf I der Blutbehandlungsvorrichtung 200 geschaltet. Die Figuren 3A und 3B zeigen die in den extrakorporalen Blutkreislauf I geschaltete Vorrichtung 100 zur Flussumkehr.

Der erste Ventilkörper 104 der Vorrichtung zur Flussumkehr weist einen ersten patientenseitigen Anschluss 110 und einen zweiten patientenseitigen Anschluss 112 auf, wobei an den ersten patientenseitigen Anschluss 110 ein erster Schlauchleitungsabschnitt 110A angeschlossen ist, der mit dem Patienten verbunden ist und an den zweiten patientenseitigen Anschluss 112 ein zweiter Schlauchleitungsabschnitt 112A der Blutleitung 110A, 112A angeschlossen ist, der mit dem Patienten verbunden ist. Der zweite Ventilkörper 102 weist einen ersten maschinenseitigen Anschluss 106 und einen zweiten maschinenseitigen Anschluss 108 auf, wobei an den ersten maschinenseitigen Anschluss ein erster Schlauchleitungsabschnitt 106A angeschlossen ist, der mit dem Auslass 204A der Blutbehandlungseinheit 204, insbesondere des Dialysators, der extrakorporalen Blutbehandlungsvorrichtung 200 verbunden ist und an den zweiten maschinenseitigen Anschluss 108 ein zweiter Schlauchleitungsabschnitt 108A der Blutleitung angeschlossen ist, der mit dem Einlass 204B der Blutbehandlungseinheit 204A verbunden ist. Neben der Blutbehandlungseinheit 204 verfügt die Blutbehandlungsvorrichtung 200 über eine Pumpe 202, die in den zu dem Einlass 204B der Blutbehandlungseinheit 204 führenden Schlauchleitungsabschnitt 108A der Blutleitung geschaltet ist. Fig. 3A zeigt den extrakorporalen Blutkreislauf I der Blutbehandlungsvorrichtung 200, wobei der Blutfluss nicht umgekehrt ist, während Fig. 3B die Blutbehandlungsvorrichtung mit der Vorrichtung zur Flussumkehr zeigt, bei der der Blutfluss umgekehrt ist.

Der zweite Ventilkörper 102 ist als kappenförmiger Körper ausgebildet, während der erste Ventilkörper 104 als Einsatzstück ausgebildet ist. Der erste Ventilkörper sitzt in dem zweiten Ventilkörper, wobei beide Ventilkörper um eine zentrale Achse 114 zwischen einer ersten Position und einer zweiten Position gegeneinander verdrehbar sind. An den Enden der Schlauchleitungsabschnitte der Blutleitung sind passende Anschlussstücke 106B, 108B, 110B, 112B vorgesehen, mit denen sich die Schlauchleitungsabschnitte einfach an die entsprechenden Anschlussstücke 106, 108, 110, 112 der Vorrichtung 100 zur Flussumkehr anschließen und wieder lösen lassen.

Fig. 3A zeigt die erste Position, in der eine Flüssigkeitsverbindung zwischen dem ersten maschinenseitigen Anschluss 106 und dem ersten patientenseitigen Anschluss 110 und eine Flüssigkeitsverbindung zwischen dem zweiten maschinenseitigen Anschluss 108 und dem zweiten patientenseitigen Anschluss hergestellt ist. Wenn der erste Ventilkörper gegenüber dein zweiten Ventilkörper um 180° verdreht wird, ist eine Flüssigkeitsverbindung zwischen dem ersten maschinenseitigen Anschluss 106 und dem zweiten patientenseitigen Anschluss 112 und eine Flüssigkeitsverbindung zwischen dem zweiten maschinenseitigen Anschluss 108 und dem ersten patientenseitigen Anschluss 110 hergestellt (Fig. 3B), so dass die Flussrichtung im extrakorporalen Blutkreislauf I umgekehrt ist.

Für den Benutzer ist es also möglich, durch Verdrehen der beiden Ventilkörper die Flussrichtung im Blutkreislauf vorzugeben. Die Vorrichtung 100 zur Flussumkehr sieht vorzugsweise vor, dass die beiden Ventilkörper 102, 104 in der ersten und zweiten Position einrastend festgelegt sind.

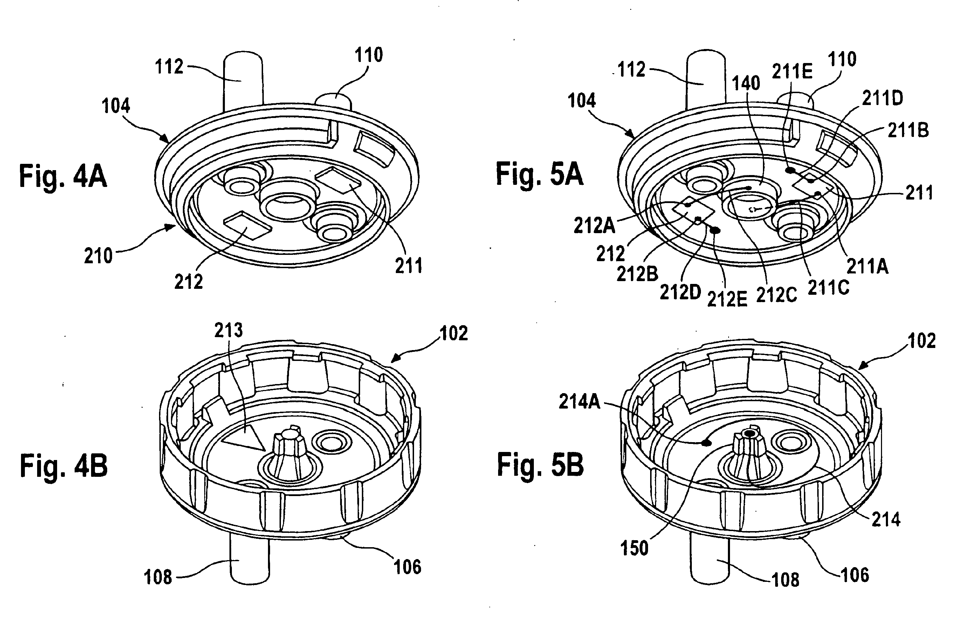

Die Figuren 4A und 4B zeigen in einer Explosionsdarstellung eine erste Ausführungsform der erfindungsgemäßen Vorrichtung 100 zur Flussumkehr, die über eine Kommunikationseinrichtung 210 verfügt, die mit einer Leseeinrichtung 270 der Blutbehandlungsvorrichtung 200 kommuniziert. Die Leseeinrichtung 270, die über eine Datenleitung 215 mit der zentralen Steuereinheit 230 der Dialysevorrichtung verbunden ist, erzeugt ein elektromagnetisches Hochfrequenzfeld im Nahbereich der Blutbehandlungsvorrichtung, das die Kommunikationseinrichtung 210 der Vorrichtung 100 zur Flussumkehr empfängt. Die Kommunikationseinrichtung 210 und die Leseeinrichtung 270 bilden zusammen ein RFID-System zur Identifikation der Flussrichtung im extrakorporalen Blutkreislauf I.

Die Kommunikationseinrichtung 210 verfügt über einen ersten RFID-Transponder 211 und einen zweiten RFID-Transponder 212, die in den Figuren nur schematisch dargestellt sind. Beide Transponder 211, 212 weisen eine nicht dargestellte Antenne und einen nicht dargestellten Transceiver sowie einen permanenten Speicher und weitere Schaltkreise auf. Da dem Fachmann Aufbau und Funktionsweise eines RFID-Transponders bekannt ist, erübrigt sich eine detaillierte Beschreibung.

Die beiden RFID-Transponder 211, 212, die eine sehr geringe Baugröße haben, sind an der Innenseite des ersten Ventilkörpers 104 einander gegenüberliegend angeordnet. Sie liegen auf einer Achse, die senkrecht zu der Achse 114 verläuft, um die sich beide Ventilkörper verdrehen lassen. Die Achse, auf der die Transponder liegen, verläuft senkrecht zu der Achse, die durch die beiden maschinenseitigen Anschlüsse verläuft. Damit sind die Transponder um 90° zu den Anschlüssen versetzt angeordnet.

Die in dem ersten Ventilkörper 104 angeordneten RFID-Transponder 211, 212 werden von dem zweiten Ventilkörper 102 elektrisch abgeschirmt, solange sich die beiden Ventilkörper nicht in einer der beiden Positionen befinden. Zur elektrischen Abschirmung ist der zweite Ventilkörper 102 an der dem ersten Ventilkörper 104 zugewandten Innenseite metallisiert. Die Metallisierung zur elektrischen Abschirmung erstreckt sich aber nicht über die gesamte Innenseite. Vielmehr bleibt ein kreissegmentförmiger Sektor 213 von der Metallisierung frei. Dieser kreissegmentförmige Sektor ist gegenüber den maschinenseitigen Anschlüssen 106, 108 des zweiten Ventilkörpers 102 um 90° versetzt angeordnet und liegt auf der Kreisbahn, auf der sich die beiden Transponder 211, 212 des ersten Ventilkörpers 104 bewegen, wenn beide Ventilkörper gegeneinander verdreht werden.

Die Transponder 211, 212 und der von der Abschirmung freie Bereich 213 sind derart angeordnet, dass in der ersten Position der nicht abgeschirmte Bereich 213 dem ersten Transponder 211 gegenüberliegt, so dass der erste Transponder 211 aktiv, der zweite Transponder 212 aber elektrisch abgeschirmt ist. In der zweiten Position ist hingegen der zweite Transponder 212 aktiv, während der erste Transponder 211 elektrisch abgeschirmt ist.

Zur Abschirmung kann auch eine Metallisierung an der Außenseite oder an beiden Seiten vorgesehen sein. Anstelle einer Metallisierung an der Oberfläche des Ventilkörpers kann der Ventilkörper auch selbst aus einem für das elektromagnetische Hochfrequenzfeld der Leseeinrichtung undurchlässigen Material bestehen. Zur besseren elektrischen Abschirmung ist auch der zweite Ventilkörper vorzugsweise an der Innenseite metallisiert. Diese Metallisierung kann sich aber vorzugsweise über die gesamte Innenseite des zweiten Ventilkörpers erstrecken.

Bei den beiden Transpondern handelt es sich vorzugsweise um passive Transponder, die ihre Energie von dem elektrischen Hochfrequenzfeld der Leseeinrichtung 270 beziehen. Wenn sich beide Ventilkörper in der ersten Position befinden, liest die Leseeinrichtung 270 von dem ersten RFID-Transponder 212 eine die erste Position kennzeichnende Kennung aus, während die Leseeinrichtung 270 eine die zweite Position kennzeichnende Kennung von dem zweiten Transponder 212 ausliest, wenn sich beide Ventilkörper in der zweiten Position befinden. Beispielsweise ist die erste Kennung eine Kennung, die sich aus einer Seriennummer des RFID-Transponders sowie einer Kennzeichnung "1" zusammensetzt, während die zweite Kennung eine Kennung ist, die sich aus der Seriennummer des zweiten Transponders und einer Kennzeichnung "2" zusammensetzt. Vorzugsweise haben beide Transponder eine gemeinsame Seriennummer, so dass sich die Transponder von den Transpondern anderer Vorrichtungen zur Flussumkehr unterscheiden lassen, die sich ebenfalls im Nahfeld der Leseeinrichtung befinden können.

Das Auslesen der die Flussrichtung im extrakorporalen Blutkreislauf kennzeichnenden Kennung kann mit der Leseeinrichtung während der Blutbehandlung kontinuierlich oder in bestimmten Zeitabständen erfolgen.

Die Fig. 5A und 5B zeigen eine zweite Ausführungsform der erfindungsgemäßen Vorrichtung zur Flussumkehr, die sich von der ersten Ausführungsform dadurch unterscheidet, dass die Aktivierung bzw. die Deaktivierung der beiden RFID-Transponder nicht durch eine elektrische Abschirmung, sondern durch die Verbindung des Transceivers nur einer der beiden Transponder mit einer gemeinsamen Antenne erfolgt. Die einander entsprechenden Teile sind daher wieder mit den gleichen Bezugszeichen versehen.

Die beiden RFID-Transponder 211, 212 verfügen nicht wie bei dem ersten Ausführungsbeispiel über eine interne Antenne, die Teil der RFID-tags sind, sondern eine gemeinsame externe Antenne 214, die jeweils mit dem nicht dargestellten Transceiver des einen oder anderen Transponders, 211, 212 verbunden wird. Die externe Antenne 214 ist eine spiralförmige Leiterbahn, die an der dem ersten Ventilkörper 104 zugewandten Innenseite des zweiten Ventilkörpers 102 angeordnet ist. Bei dieser Ausführungsform schirmen die Ventilkörper die Transponder nicht elektrisch ab.

Die beiden RFID-Transponder 211, 212 weisen jeweils zwei Anschlüsse 211A, 212A bzw. 211B, 212B für die Antenne auf. Der jeweils erste Anschluss 211A, 212A der beiden Transponder 211, 212 ist jeweils über eine elektrische Verbindungsleitung 211C, 212C mit einem zentralen hohlzylindrischen Ansatz 140 des ersten Ventilkörpers 104 verbunden, der einen zentralen vorspringenden Ansatz 150 des zweiten Ventilkörpers 102 umschließt, wenn beide Ventilkörper zusammengesteckt sind (Fig. 2). Der zylindrische Ansatz 140 des ersten Ventilkörpers 104 und der vorspringende Ansatz 150 des zweiten Ventilkörpers 102 sind jeweils metallisiert, so dass eine elektrische Verbindung zwischen beiden Ventilkörpern hergestellt ist.

Der jeweils zweite Anschluss 211B, 212B der beiden Transponder 211, 212 ist über eine elektrische Verbindungsleitung 211D, 212D jeweils mit einem elektrischen Kontakt 211E, 212E verbunden. Die beiden elektrischen Kontakte 211E und 212E sind auf der dem zweiten Ventilkörper 102 zugewandten Innenseite des ersten Ventilkörpers 110 einander gegenüberliegend angeordnet. Sie liegen auf einer Achse, die senkrecht zu der Drehachse 114 verläuft. Die Achse, auf der die beiden Kontaktpunke liegen, verläuft im rechten Winkel zu der Achse, auf der die patientenseitigen Anschlüsse 102, 108 des ersten Ventilkörpers liegen, d.h. die Kontakte sind um 90° zu den Anschlüssen versetzt angeordnet.

Der erste Anschluss der gemeinsamen Antenne 214 ist elektrisch mit dem vorspringenden Ansatz 150 des zweiten Ventilkörpers 104 verbunden, so dass eine permanente elektrische Verbindung mit den beiden ersten Anschlüssen 211A, 212A der beiden Transponder 211, 212 hergestellt ist. Der zweite Anschluss der Antenne 214 ist elektrisch mit einem Schleifkontakt 214A verbunden, der auf der dem ersten Ventilkörper 104 zugewandten Innenseite des zweiten Ventilkörpers 102 angeordnet ist.

Wenn sich beide Ventilkörper 102, 104 in der ersten Position befinden, kontaktiert der Schleifkontakt 214A des zweiten Ventilkörpers 102 den ersten Kontakt 211E des ersten Ventilkörpers 104, während in der zweiten Position der Schleifkontakt 214A den zweiten Kontakt 212E des zweiten Ventilkörpers 104 kontaktiert. Folglich ist in der ersten Position nur der nicht explizit dargestellte Transceiver des ersten Transponders 211 elektrisch mit der Antenne 214 verbunden, während in der zweiten Position nur der nicht explizit dargestellte zweite Transceiver des zweiten Transponders 212 mit der Antenne 214 elektrisch verbunden ist.

Wie bei dem ersten Ausführungsbeispiel kann die Leseeinrichtung 270 in der ersten Position nur die Kennung des ersten und in der zweiten Position nur die Kennung des zweiten Transponders auslesen. Dadurch ist eine eindeutige Identifikation der Flussrichtung möglich.

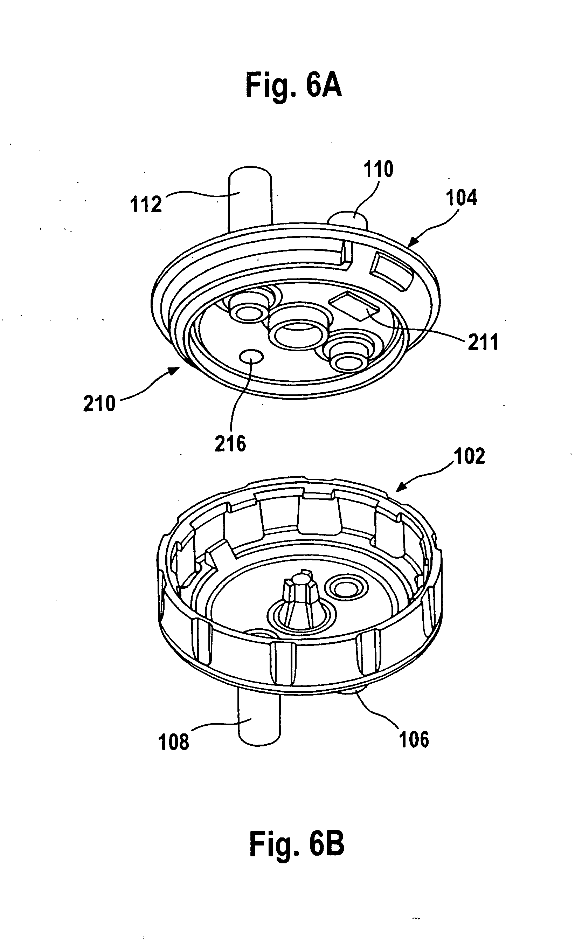

Die Figuren 6A und 6B zeigen in vereinfachter schematischer Darstellung eine weitere Ausführungsform der erfindungsgemäßen Vorrichtung zur Flussumkehr, die sich von den beiden anderen Ausführungsformen dadurch unterscheidet, dass die Kommunikationseinrichtung nur über einen einzigen RFID-Transponder verfügt. Die einander entsprechenden Teile sind wieder mit den gleichen Bezugszeichen versehen.

Bei der alternativen Ausführungsform der Figuren 6A und 6B weist die Kommunikationseinrichtung 210 eine die erste und zweite Position der Ventilkörper 102, 104 detektierende Einrichtung 216 auf, die nur schematisch dargestellt ist. Bei einer derartigen Einrichtung zur Positionserkennung der Ventilkörper 102, 104 kann es sich beispielsweise um einen positionsabhängigen Schalter oder resistive oder kapazitive Winkelsensoren handeln. Allein entscheidend ist, dass die Einrichtung zur Positionserkennung 216 für den einzigen RFID-Transponder 211 ein Signal erzeugt, das für die erste oder zweite Position kennzeichnend ist. Der einzige RFID-Transponder hat bei dieser Ausführungsform zwei Kennungen für die beiden Positionen der Ventilkörper, die von der Leseeinrichtung 270 ausgelesen werden.

Bei der extrakorporalen Blutbehandlungsvorrichtung kann es sich um eine konventionelle Blutbehandlungsvorrichtung, beispielsweise eine Dialysevorrichtung handeln, die neben den bekannten Komponenten über die erfindungsgemäße Leseeinrichtung 270 verfügt. Die automatische Identifikation der Flussrichtung im extrakorporalen Blutkreislauf I ist insbesondere dann von Vorteil, wenn die Blutbehandlungsvorrichtung über eine Einrichtung 240 zur Bestimmung eines Parameters der Blutbehandlung, beispielsweise der Clearance verfügt, die eine Messung einer charakteristischen Größe des Bluts vor und nach der Umkehr des Blutflusses erfordert. Derartige Einrichtungen zur Bestimmung von Parametern der Blutbehandlung gehören zum Stand der Technik.

In den Figuren 3A und 3B ist die Einrichtung 240 zur Bestimmung eines Parameters der Blutbehandlung nur schematisch angedeutet. Die Einrichtung 240 zur Bestimmung des Parameters der Blutbehandlung ist mit der zentralen Steuereinheit 230 der Blutbehandlungsvorrichtung über eine Datenleitung 241 verbunden. Neben der Steuerung der einzelnen Aggregate der Blutbehandlungsvorrichtung übernimmt die zentrale Steuereinheit 230 auch die Steuerung der Leseeinrichtung 270 und der Messeinrichtung 240.

Darüber hinaus verfügt die Blutbehandlungsvorrichtung 200 über eine Alarmeinheit 250, die über eine Datenleitung 251 mit Steuereinheit 230 verbunden ist sowie über eine Anzeigeeinheit 260, die ebenfalls über eine Datenleitung 261 mit der Steuereinheit verbunden ist.

Die Bedienungsführung der Blutbehandlungsvorrichtung sieht vor, dass für die Durchführung einer Messung einer charakteristischen Größe vor und nach der Umkehr des Blutflusses zur Bestimmung eines Parameters der Blutbehandlung der Benutzer aufgefordert wird, durch manuelles Verdrehen der beiden Ventilkörper 102, 104 der Vorrichtung 100 zur Umkehr des Blutflusses den Blutfluss im extrakorporalen Blutkreislauf umzukehren. Diese Aufforderung wird dem Benutzer auf der Anzeigeeinheit 260 signalisiert.

Nach der Durchführung der ersten Messung mit normalem Blutfluss überprüft die Leseeinrichtung 270, ob der Benutzer, der zur Umkehr des Blutflusses aufgefordert worden ist, durch Verdrehen der Ventilkörper den Blutfluss umgekehrt hat. Wenn der Benutzer dieser Aufforderung nicht nachgekommen ist, stellt dies die Leseeinrichtung 270 dadurch fest, dass nicht die Kennung für die zweite, sondern für die erste Position der Ventilkörper ausgelesen wird. Dann erzeugt die Leseeinrichtung 270 ein Alarmsignal, das die Alarmeinheit 250 über die Steuereinheit 230 empfängt. Die Alarmeinheit 250 gibt dann einen akustischen und/oder optischen Alarm als erneute Aufforderung für den Benutzer den Blutfluss umzukehren.

Nach Durchführung der zweiten Messung mit umgekehrtem Blutfluss wird der Benutzer auf der Anzeigeeinheit 260 wieder zur Umkehrung des Blutflusses aufgefordert. Die Leseeinrichtung 270 überprüft dann wieder, ob der Blutfluss in der ursprünglichen Richtung erfolgt. Ansonsten wird wieder Alarm gegeben. Die richtige Position stellt die Leseeinrichtung 270 dadurch fest, dass nicht die Kennung für die zweite, sondern für die erste Position der Ventilkörper 102, 104 ausgelesen wird.

Die Routine zur Durchführung der Messung wird vorzugsweise unterbrochen, wenn die Leseeinrichtung 270 nicht die korrekte Flussrichtung ausgelesen hat. Dadurch werden nicht nur fehlerhafte Messungen vermieden, sondern es wird auch sichergestellt, dass die extrakorporale Blutbehandlung nach den Messungen nicht mit einem umgekehrten Blutfluss betrieben wird. Mit der Leseeinrichtung 270 ist es nicht nur möglich, die Flussrichtung zu detektieren, sondern auch festzustellen, ob sich die Ventilkörper 102, 104 in der korrekten eingerasteten Position befinden, in denen eine Flüssigkeitsverbindung zwischen den Anschlüssen hergestellt ist. Beispielsweise könnte die Gefahr bestehen, dass bei der Vorrichtung zur Flussumkehr, die aus der WO 2006/042016 A2 bekannt ist, die Ventilkörper nicht in einer der beiden Positionen eingerastet sind, sondern gegenüber der Rastposition verdreht sind. In diesem Fall wären beide Transponder 211, 212 nicht aktiv, was von der Leseeinrichtung 270 dadurch erkannt wird, dass die entsprechende Kennung nicht ausgelesen wird. Dies ist auch dann der Fall, wenn zwar eine Flüssigkeitsverbindung hergestellt ist, die Lumen der einander gegenüberliegenden Anschlüsse aber nicht genau fluchten, so dass nur ein enger Spalt für den Durchfluss des Bluts verbleibt. Da dies zu einer Blutschädigung durch Hämolyse führen könnte, sieht die erfindungsgemäße Blutbehandlungsvorrichtung 200 vorzugsweise die Unterbrechung des Blutflusses vor, wenn die Leseeinrichtung 270 eine ordnungsgemäße Ausrichtung der Ventilkörper zueinander in den beiden Positionen nicht erkennt. Dadurch wird die Sicherheit der Blutbehandlung sowie der Messungen weiter erhöht.

In der Praxis stellt sich darüber hinaus das Problem, dass mehrere Blutbehandlungsvorrichtungen in enger Nachbarschaft betrieben werden. Daher ist eine Identifikation derjenigen Vorrichtung zur Umkehr des Blutflusses erforderlich, die der jeweiligen Blutbehandlungsvorrichtung zugeordnet ist.

Eine automatische Identifikation erfolgt bei der erfindungsgemäßen Blutbehandlungsvorrichtung 200 mit einem Funktionstest der Vorrichtung 100 zur Umkehrung des Blutflusses. Der Benutzer wird nach der Erkennung eines oder mehrerer Vorrichtungen zur Flussumkehr von der Blutbehandlungsvorrichtung 200 auf der Anzeigeeinheit 260 aufgefordert, die Ventilkörper 102, 104 der Vorrichtung 100 zur Flussumkehr gegeneinander zu verdrehen, die der jeweiligen Blutbehandlungsvorrichtung zugeordnet ist. Dadurch werden innerhalb eines vorgegebenen Zeitintervalls, das der Benutzer zum Verdrehen der Ventilkörper benötigt, die beiden RFID-Transponder 211, 212 nacheinander aktiviert. Die Leseeinrichtung 270 der Blutbehandlungsvorrichtung erkennt daraufhin die zugehörige Vorrichtung zur Flussumkehr dadurch, dass innerhalb des vorgegebenen Zeitintervalls ein Wechsel zwischen der die beiden Positionen kennzeichnenden Kennung stattfindet. Hierfür verfügt die Leseinrichtung über entsprechende Mittel. Die gemeinsame Seriennummer der beiden Kennungen für die Flussrichtung, die von der Leseeinrichtung ausgelesen werden, erlaubt nunmehr eine Identifikation der zugehörigen Vorrichtung zur Flussumkehr.

Die Menüführung der Dialysevorrichtung kann weiterhin vorsehen, dass allein durch die Erkennung der zugehörigen Vorrichtung zur Flussumkehr die Messungen für die Bestimmung eines Parameters der Blutbehandlung automatisch durchgeführt werden, ohne dass es einer weiteren Eingabe durch den Benutzer bedarf.

Die erfindungsgemäße Blutbehandlungsvorrichtung bietet zusammen mit der Vorrichtung zur Flussumkehr noch weitere Vorteile, die nachfolgend erläutert werden.

Neben nativen Fisteln und PTFE-Grafts werden gerade bei schwierigen Gefäßbedingungen doppellumige zentralvenöse Katheter als Gefäßzugang verwendet. Bei diesen werden arterieller und venöser Blutschlauch an zwei verschiedene Schenkel des Katheters angeschlossen. Zur Vermeidung von Rezirkulation sind die beiden im Vorhof fixierten Enden des Katheters räumlich (ca. 2cm) voneinander getrennt. Bei einem voll funktionstüchtigen Katheter erfolgt die Blutentnahme (arteriell) durch das kürzere der beiden Enden, die Rückgabe durch das längere Ende (venös). Gelegentlich kann es dazu kommen, dass der gewünschte Blutfluss in dieser Konfiguration nicht erreicht wird, wobei die Gründe hierfür nicht vollständig geklärt sind (s. Depner, "Catheter Performance", Seminars in Dialysis 14/6 (2001), Seite 425-431). In diesen Fällen werden typischerweise vom Pflegepersonal die Anschlüsse am Katheter vertauscht, wodurch oft der gewünschte Fluss wieder erreicht werden kann. In der Literatur finden sich Angaben, dass eine Behandlung, wobei die Flussrichtung vertauscht ist, in bis zu 30 % der Fälle stattfindet (N. Pannu et al., "Optimizing dialysis delivery in tunneled dialysis catheters", ASAIO Journal 52/2 (2006), Seite 57-162). Das Vertauschen führt jedoch zu einer erhöhten Access-Rezirkulation, was zu einer Verminderung der Dialyseeffizienz führt.

Durch Verwendung der erfindungsgemäßen Vorrichtung zur Flussumkehr im Blutschlauchsystem kann nun zum einen das Vertauschen leichter und mit geringerem Infektionsrisiko erfolgen. Zum anderen ist durch die selbständige Positionserkennung über das RFID-Lesegerät im Dialysegerät eine automatische Protokollierung der Orientierung der Blutzugangsschläuche im Dialysegerät möglich. Hierdurch können Behandlungen mit Zugangsproblemen automatisch registriert werden. Wird zudem bei einer Messung der Clearance eine zu niedrige Clearance erkannt, so kann das Vertauschen der Anschlüsse des Kathethers zugleich als eine Ursache für die Messung der zu niedrigen Clearance erkannt und somit von anderen möglichen Ursachen, z.B. Clotting des Dialysators, unterschieden werden.

Die in den Figuren dargestellten Ausführungsformen der Erfindung stellen nur Ausführungsbeispiele dar, mit denen eine besonders bevorzugte Verwendung der Erfindung in dem mechanischen Umschaltventil gezeigt werden soll, das aus der WO 2006/042016 A2 bekannt ist. Das mechanische Umschaltventil als solches kann aber auch anderes ausgebildet sein, als in der WO 2006/042016 A2 beschrieben ist.

## Patentansprüche

1. Vorrichtung zur Umkehr des Blutflusses für eine extrakorporale Blutbehandlungsvorrichtung, die einen extrakorporalen Blutkreislauf umfasst, der eine von einem Patienten abgehende zu einer Blutbehandlungseinheit führende arterielle Blutleitung und eine von der Blutbehandlungseinheit abgehende zu dem Patienten führende venöse Blutleitung aufweist,
wobei die Vorrichtung zur Flussumkehr aufweist:
einen ersten Ventilkörper (104), der einen ersten patientenseitigen Anschluss (110) für einen ersten patientenseitigen Schlauchleitungsabschnitt der Blutleitung und einen zweiten patientenseitigen Anschluss (112) für einen zweiten patientenseitigen Schlauchleitungsabschnitt der Blutleitung aufweist,
einen zweiten Ventilkörper (102), der einen ersten maschinenseitigen Anschluss (106) für einen ersten maschinenseitigen Schlauchleitungsabschnitt der Blutleitung und einen zweiten maschinenseitigen Anschluss (108) für einen zweiten maschinenseitigen Schlauchleitungsabschnitt der Blutleitung aufweist,
wobei der erste und zweite Ventilkörper (104, 102) derart um eine Achse (144) drehbar ausgebildet sind, dass
in einer ersten Position eine Flüssigkeitsverbindung zwischen dem ersten patientenseitigen Anschluss (110) und dem ersten maschinenseitigen Anschluss (106) einerseits und zwischen dem zweiten patientenseitigen Anschluss (112) und dem zweiten maschinenseitigen Anschluss (108) andererseits hergestellt wird und
in einer zweiten Position eine Flüssigkeitsverbindung zwischen dem ersten patientenseitigen Anschluss (110) und dem zweiten maschinenseitigen Anschluss (108) einerseits und zwischen dem zweiten patientenseitigen Anschluss (112) und dem ersten maschinenseitigen Anschluss (106) andererseits hergestellt wird,
**dadurch gekennzeichnet, dass**
die Vorrichtung zur Flussumkehr aufweist:
eine Kommunikationseinrichtung (210) zum Empfangen eines elektromagnetischen Hochfrequenzfeldes von einer Leseeinrichtung der extrakorporalen Blutbehandlungsvorrichtung und zum Erzeugen mindestens einer die Position der Ventilkörper kennzeichnenden Kennung, die von der Leseeinrichtung auslesbar ist, wobei
die Kommunikationseinrichtung (210) einen ersten RFID-Transponder (211) und einen zweiten RFID-Transponder (212) aufweist, wobei der erste RFID-Transponder eine die erste Position kennzeichenden Kennung und der zweite RFID-Transponder eine die zweite Position kennzeichnende Kennung sendet.

2. Vorrichtung nach Anspruch 1, **dadurch gekennzeichnet, dass** der erste RFID-Transponder (211) an einer die erste Position kennzeichnenden Stelle an dem ersten oder zweiten Ventilkörpers angeordnet ist und der zweite RFID-Transponder (212) an einer die zweite Position kennzeichnenden Stelle an dem ersten oder zweiten Ventilkörper (104, 102) angeordnet ist,
und dass der erste und/oder zweite Ventilkörper (104, 102) derart elektrisch abgeschirmt ist, dass in der ersten Position nur der erste RFID-Transponder (211) zum Senden der die erste Position kennzeichnenden Kennung von der Leseeinrichtung auslesbar ist und in der zweiten Position nur der zweite RFID-Transponder (212) zum Senden der die zweite Position kennzeichnenden Kennung von der Leseeinrichtung auslesbar ist.

3. Vorrichtung nach Anspruch 2, **dadurch gekennzeichnet, dass** der erste und zweite RFID-Transponder (211, 212) an der Innenseite des ersten Ventilkörpers (104) auf einer senkrecht zur Drehachse der Ventilkörper liegenden Achse einander gegenüberliegend angeordnet sind, wobei die Innenseite des zweiten Ventilkörpers bis auf einen Ausschnitt (213) zur Abschirmung des RFID-Transponders (211, 212) metallisiert ist, der in der ersten Position dem ersten RFID-Transponder (211) und in der zweiten Position dem zweiten RFID-Transponder (212) gegenüberliegt, so dass in der ersten Position nur der erste RFID-Transponder zum Senden der die erste Position kennzeichnenden Kennung von der Leseeinrichtung auslesbar ist und in der zweiten Position nur der zweite RFID-Transponder zum Senden der die zweite Position kennzeichnenden Kennung von der Leseeinrichtung auslesbar ist.

4. Vorrichtung nach einem der Ansprüche 1 bis 3, **dadurch gekennzeichnet, dass** die Innenseite des ersten Ventilkörpers (104) zur elektrischen Abschirmung der RFID-Transponder (211, 212) metallisiert ist.

5. Vorrichtung nach Anspruch 1, **dadurch gekennzeichnet, dass** in der ersten Position nur der Tranceiver des ersten RFID-Transponders (211) mit einer Antenne (214) verbunden ist und in der zweiten Position nur der Transceiver des zweiten RFID-Transponders (212) mit einer Antenne (214) verbunden ist, so dass in der ersten Position nur der erste RFID-Transponder zum Senden der die erste Position kennzeichnenden Kennung von der Leseeinrichtung auslesbar ist und in der zweiten Position nur der zweite RFID-Transponder zum Senden der die zweite Position kennzeichnenden Kennung von der Leseeinrichtung auslesbar ist.

6. Vorrichtung nach Anspruch 5, **dadurch gekennzeichnet, dass** die Tranceiver des ersten und zweiten RFID-Transponders (211) in dem ersten Ventilkörper (104) angeordnet sind und in dem zweiten Ventilkörper (212) eine gemeinsame Antenne (214) für die Tranceiver des ersten und zweiten RFID-Transponders angeordnet ist, wobei eine Kontakteinrichtung (214A; 211 E, 212E) zur Herstellung einer elektrischen Verbindung zwischen dem ersten Tranceiver und der Antenne in der ersten Position und zur Herstellung einer elektrischen Verbindung zwischen dem zweiten Transceiver und der Antenne in der zweiten Position herstellbar ist.

7. Vorrichtung nach Anspruch 6, **dadurch gekennzeichnet, dass** die Kontakteinrichtung eine erste elektrische Verbindung (211C, 212C; 140, 150) zur Herstellung einer dauerhaften elektrischen Verbindung zwischen dem ersten und zweiten Transceiver und der Antenne (214) aufweist, wobei die Kontakteinrichtung an dem ersten Ventilkörper (104) einen mit dem ersten Tranceiver verbundenen Kontakt (211 E) und einen mit dem zweiten Tranceiver Kontakt (212E) und an dem zweiten Ventilkörper (102) einen Schleifkontakt (214A) aufweist, wobei der Schleifkontakt in der ersten Position mit dem ersten Kontakt und in der zweiten Position mit dem zweiten Kontakt kontaktierbar ist.

8. Vorrichtung nach einem der Ansprüche 1 bis 7, **dadurch gekennzeichnet, dass** der erste und zweite RFID-Transponder (211, 212) passive Transponder sind.

9. Vorrichtung zur Umkehr des Blutflusses für eine extrakorporale Blutbehandlungsvorrichtung, die einen extrakorporalen Blutkreislauf umfasst, der eine von einem Patienten abgehende zu einer Blutbehandlungseinheit führende arterielle Blutleitung und eine von der Blutbehandlungseinheit abgehende zu dem Patienten führende venöse Blutleitung aufweist,
wobei die Vorrichtung zur Flussumkehr aufweist:
einen ersten Ventilkörper (104), der einen ersten patientenseitigen Anschluss (110) für einen ersten patientenseitigen Schlauchleitungsabschnitt der Blutleitung und einen zweiten patientenseitigen Anschluss (112) für einen zweiten patientenseitigen Schlauchleitungsabschnitt der Blutleitung aufweist,
einen zweiten Ventilkörper (102), der einen ersten maschinenseitigen Anschluss (106) für einen ersten maschinenseitigen Schlauchleitungsabschnitt der Blutleitung und einen zweiten maschinenseitigen Anschluss (108) für einen zweiten maschinenseitigen Schlauchleitungsabschnitt der Blutleitung aufweist,
wobei der erste und zweite Ventilkörper (104, 102) derart um eine Achse (144) drehbar ausgebildet sind, dass
in einer ersten Position eine Flüssigkeitsverbindung zwischen dem ersten patientenseitigen Anschluss (110) und dem ersten maschinenseitigen Anschluss (106) einerseits und zwischen dem zweiten patientenseitigen Anschluss (112) und dem zweiten maschinenseitigen Anschluss (108) andererseits hergestellt wird und
in einer zweiten Position eine Flüssigkeitsverbindung zwischen dem ersten patientenseitigen Anschluss (110) und dem zweiten maschinenseitigen Anschluss (108) einerseits und zwischen dem zweiten patientenseitigen Anschluss (112) und dem ersten maschinenseitigen Anschluss (106) andererseits hergestellt wird,
**dadurch gekennzeichnet, dass**
die Vorrichtung zur Flussumkehr aufweist:
eine Kommunikationseinrichtung (210) zum Empfangen eines elektromagnetischen Hochfrequenzfeldes von einer Leseeinrichtung der extrakorporalen Blutbehandlungsvorrichtung und zum Erzeugen mindestens einer die Position der Ventilkörper kennzeichnenden Kennung, die von der Leseeinrichtung auslesbar ist, wobei
die Kommunikationseinrichtung (210) einen RFID-Transponder (211) aufweist, der eine die erste Position kennzeichnende Kennung und eine die zweite Position kennzeichnende Kennung sendet.

10. Vorrichtung nach Anspruch 9, **dadurch gekennzeichnet, dass** eine die erste und zweite Position der Ventilkörper (104, 102) detektierende Einrichtung (216) vorgesehen ist, die derart mit dem RFID-Transponder (211) zusammenwirkt, dass von der Leseeinrichtung in der ersten Position die die erste Position kennzeichnende Kennung auslesbar ist und in der zweiten Position die die zweite Position kennzeichnende Kennung auslesbar ist.

11. Vorrichtung nach Anspruch 9 oder 10, **dadurch gekennzeichnet, dass** der RFID-Transponder (211) ein passiver Transponder ist.

12. Blutbehandlungsvorrichtung zur Durchführung einer extrakorporalen Blutbehandlung, bei der in einem extrakorporalen Blutkreislauf (I) das Blut eines Patienten über eine arterielle Blutleitung (108A) zu einer Blutbehandlungseinheit (204) strömt und von der Blutbehandlungseinheit über eine venöse Blutleitung (106A) zum Patienten strömt, wobei in die Blutleitungen (106A, 108A) eine Vorrichtung (100) zur Umkehr des Blutflusses im extrakorporalen Blutkreislauf (I) nach einem der Ansprüche 1 bis 11 geschaltet ist,
**dadurch gekennzeichnet, dass** die Blutbehandlungsvorrichtung (100) eine Leseeinrichtung (270) aufweist, die derart ausgebildet ist, dass die die erste oder zweite Position kennzeichnende Kennung der Kommunikationseinrichtung (210) auslesbar ist.

13. Blutbehandlungsvorrichtung nach Anspruch 12, **dadurch gekennzeichnet, dass** die Blutbehandlungsvorrichtung eine Einrichtung (240) zur Bestimmung eines Parameters der Blutbehandlung auf der Grundlage einer ersten Messung einer charakteristischen Größe vor und einer zweiten Messung einer charakteristischen Größe nach der Umkehr des Blutflusses aufweist, wobei die Blutbehandlungsvorrichtung eine Alarmeinheit (250) aufweist, die derart mit der Einrichtung (240) zur Bestimmung eines Parameters der Blutbehandlung zusammenwirkt, dass die Alarmeinheit (250) einen Alarm gibt, wenn nach der ersten Messung die Leseinrichtung (270) nicht die die zweite Position kennzeichnende Kennung und nach der zweiten Messung die Leseinrichtung nicht die die erste Position kennzeichnende Kennung empfangen hat.

14. Blutbehandlungsvorrichtung nach Anspruch 12 oder 13, **dadurch gekennzeichnet, dass** die Blutbehandlungsvorrichtung eine Einrichtung (220) zur Erkennung der Kommunikationseinrichtung der zugehörigen Vorrichtung zur Umkehr des Blutflusses aufweist, wobei die Einrichtung (220) zur Erkennung der Kommunikationseinrichtung Mittel aufweist, die derart ausgebildet sind, dass ein Wechsel zwischen der die erste und zweite Position kennzeichnenden Kennung innerhalb eines vorgegebenen Zeitintervalls erkannt wird.

15. Verfahren zur Feststellung der Umkehr des Blutflusses bei einer extrakorporalen Blutbehandlung mit einer extrakoporalen Blutbehandlungsvorrichtung, die eine Vorrichtung zur Umkehr des Blutflusses im extrakorporalen Blutkreislauf aufweist, **dadurch gekennzeichnet, dass** von einer Leseeinrichtung (270) der Blutbehandlungsvorrichtung ein elektromagnetisches Hochfrequenzfeld ausgesandt wird, das von einer Kommunikationseinrichtung (210) der Vorrichtung zur Flussumkehr empfangen wird, wobei die Kommunikationseinrichtung (210) eine die Flussrichtung im extrakorporalen Blutkreislauf in die eine Richtung kennzeichnende erste Kennung und eine die Flussrichtung im extrakorporalen Blutkreislauf in die andere Richtung kennzeichnende zweite Kennung erzeugt, die von der Leseinrichtung der Blutbehandlungsvorrichtung ausgelesen wird, und wobei die erste Kennung von einem ersten RFID-Transponder (211) und die zweite Kennung von einem zweiten RFID-Transponder (212) gesendet wird oder die erste und zweite Kennung von einem einzigen RFID-Transponder (211) gesendet wird.

## Claims

1. A device for reversing the blood flow for an extracorporeal blood treatment device which comprises an extracorporeal blood circuit which has an arterial blood line leading from a patient to a blood treatment unit, and a venous blood line leading from the blood treatment unit to the patient,
wherein the device for reversing the flow has:
a first valve body (104) which has a first patient-side connector (110) for a first patient-side flexible tube section of the blood line, and a second patient-side connector (112) for a second patient-side flexible tube section of the blood line,
a second valve body (102) which has a first machine-side connector (106) for a first machine-side flexible tube section of the blood line, and a second machine-side connector (108) for a second machine-side flexible tube section of the blood line,
wherein the first and second valve body (104, 102) are rotatably formed about an axis (144) in such a way that
in a first position, a fluid connection is made between the first patient-side connector (110) and the first machine-side connector (106) on the one hand, and between the second patient-side connector (112) and the second machine-side connector (108) on the other, and
in a second position, a fluid connection is made between the first patient-side connector (110) and the second machine-side connector (108) on the one hand, and
between the second patient-side connector (112) and the first machine-side connector (106) on the other,
**characterized in that**
the device for reversing the flow has:
a communications device (210) for receiving an electromagnetic high-frequency field from a read device of the extracorporeal blood treatment device, and for producing at least one identifier which identifies the position of the valve bodies and which can be read out by the read unit, wherein
the communications device (210) has a first RFID transponder (211) and a second RFID transponder (212), wherein the first RFID transponder transmits an identifier which identifies the first position, and the second RFID transponder transmits an identifier which identifies the second position.

2. The device as claimed in claim 1, **characterized in that** the first RFID transponder (211) is arranged at a point on the first or second valve body which identifies the first position, and the second RFID transponder (212) is arranged at a point on the first or second valve body (104, 102) which identifies the second position,
and that the first and/or second valve body (104, 102) is electrically screened in such a way that, in the first position, only the first RFID transponder (211) for transmitting the identifier which identifies the first position can be read out by the read device, and, in the second position, only the second RFID transponder (212) for transmitting the identifier which identifies the second position can be read out by the read device.

3. The device as claimed in claim 2, **characterized in that** the first and second RFID transponder (211, 212) are arranged opposite one another on the inside of the first valve body (104) on an axis which lies perpendicular to the axis of rotation of the valve body, wherein the inside of the second valve body is metalized in order to screen the RFID transponder (211, 212) apart from a cutout (213) which, in the first position, lies opposite the first RFID transponder (211) and, in the second position, lies opposite the second RFID transponder (212), so that, in the first position, only the first RFID transponder for transmitting the identifier which identifies the first position can be read out by the read device and, in the second position, only the second RFID transponder for transmitting the identifier which identifies the second position can be read out by the read device.

4. The device as claimed in one of claims 1 to 3, **characterized in that** the inside of the first valve body (104) is metalized to electrically screen the RFID transponder (211,212).

5. The device as claimed in claim 1, **characterized in that**, in the first position, only the transceiver of the first RFID transponder (211) is connected to an antenna (214) and, in the second position, only the transceiver of the second RFID transponder (212) is connected to an antenna (214), so that, in the first position, only the first RFID transponder for transmitting the identifier which identifies the first position can be read out by the read device and, in the second position, only the second RFID transponder for transmitting the identifier which identifies the second position can be read out by the read device.

6. The device as claimed in claim 5, **characterized in that** the transceivers of the first and second RFID transponder (211) are arranged in the first valve body (104), and a common antenna (214) for the transceivers of the first and second RFID transponder is arranged in the second valve body (212), wherein a contact device (214A; 211E, 212E) for making an electrical connection between the first transceiver and the antenna in the first position and for making an electrical connection between the second transceiver and the antenna in the second position can be produced.

7. The device as claimed in claim 6, **characterized in that** the contact device has a first electrical connection (211C, 212C; 140, 150) for making a permanent electrical connection between the first and second transceiver and the antenna (214), wherein, on the first valve body (104), the contact device has a contact (211E) connected to the first transceiver and a contact (212E) connected to the second transceiver and, on the second valve body (102), a sliding contact (214A), wherein the sliding contact can be connected to the first contact in the first position and to the second contact in the second position.

8. The device as claimed in one of claims 1 to 7, **characterized in that** the first and second RFID transponder (211, 212) are passive transponders.

9. A device for reversing the blood flow for an extracorporeal blood treatment device which comprises an extracorporeal blood circuit which has an arterial blood line leading from a patient to a blood treatment unit, and a venous blood line leading from the blood treatment unit to the patient,
wherein the device for reversing the flow has:
a first valve body (104) which has a first patient-side connector (110) for a first patient-side flexible tube section of the blood line, and a second patient-side connector (112) for a second patient-side flexible tube section of the blood line,
a second valve body (102) which has a first machine-side connector (106) for a first machine-side flexible tube section of the blood line, and a second machine-side connector (108) for a second machine-side flexible tube section of the blood line,
wherein the first and second valve body (104, 102) are rotatably formed about an axis (144) in such a way that
in a first position, a fluid connection is made between the first patient-side connector (110) and the first machine-side connector (106) on the one hand, and
between the second patient-side connector (112) and the second machine-side connector (108) on the other, and
in a second position, a fluid connection is made between the first patient-side connector (110) and the second machine-side connector (108) on the one hand, and
between the second patient-side connector (112) and the first machine-side connector (106) on the other,
**characterized in that**
the device for reversing the flow has:
a communications device (210) for receiving an electromagnetic high-frequency field from a read device of the extracorporeal blood treatment device, and for producing at least one identifier which identifies the position of the valve bodies and which can be read out by the read unit, wherein
the communications device (210) has an RFID transponder (211) which transmits an identifier which identifies the first position and an identifier which identifies the second position.

10. The device as claimed in claim 9, **characterized in that** a device (216) is provided which detects the first and second position of the valve bodies (104, 102) and which acts together with the RFID transponder (211) in such a way that, in the first position, the identifier which identifies the first position can be read out by the read device and, in the second position, the identifier which identifies the second position can be read out by the read device.

11. The device as claimed in claim 9 or 10, **characterized in that** the RFID transponder (211) is a passive transponder.

12. A blood treatment device for carrying out an extracorporeal blood treatment, in which, in an extracorporeal blood circuit (I), the blood of a patient flows via an arterial blood line (108A) to a blood treatment unit (204), and from the blood treatment unit via a venous blood line (106A) to the patient, wherein a device (100) for reversing the blood flow in the extracorporeal blood circuit (I) as claimed in one of claims 1 to 11 is connected in the blood lines (106A, 108A),
**characterized in that** the blood treatment device (100) has a read device (270) which is designed in such a way that the identifier which identifies the first or second position can be read out from the communications device (210).

13. The blood treatment device as claimed in claim 12, **characterized in that** the blood treatment device has a device (240) for determining a parameter of the blood treatment based on a first measurement of a characteristic quantity before and a second measurement of a characteristic quantity after the reversal of the blood flow, wherein the blood treatment device has an alarm unit (250), which acts together with the device (240) for determining a parameter of the blood treatment in such a way that the alarm unit (250) gives an alarm if, after the first measurement, the read device (270) has not received the identifier which identifies the second position and, after the second measurement, the read device has not received the identifier which identifies the first position.

14. The blood treatment device as claimed in claim 12 or 13, **characterized in that** the blood treatment device has a device (220) for detecting the communications device of the associated device for reversing the blood flow, wherein the device (220) for detecting the communications device has means which are designed in such a way that a change between the identifier which identifies the first and second position within a predetermined time interval is detected.

15. A method for determining the reversal of the blood flow during an extracorporeal blood treatment having an extracorporeal blood treatment device which has a device for reversing the blood flow in the extracorporeal blood circuit, **characterized in that** an electromagnetic high-frequency field, which is received by a communications device (210) of the device for reversing the flow, is transmitted by a read device (270) of the blood treatment device, wherein the communications device (210) produces a first identifier which identifies the flow direction in the extracorporeal blood circuit in the one direction, and a second identifier which identifies the flow direction in the extracorporeal blood circuit in the other direction and is read out by the read device of the blood treatment device, and wherein the first identifier is transmitted by a first RFID transponder (211), and the second identifier by a second RFID transponder (212), or the first and second identifier is transmitted by a single RFID transponder (211).

## Revendications

1. Dispositif pour inverser le flux sanguin pour un dispositif de traitement extracorporel du sang qui comprend une circulation sanguine extracorporelle qui présente une conduite de sang artériel partant d'un patient conduisant à une unité de traitement du sang et une conduite de sang veineux partant de l'unité de traitement du sang, conduisant au patient,
le dispositif présentant pour l'inversion du flux :
un premier corps de clapet (104) qui présente un premier raccordement (110) du côté du patient pour un premier segment tubulaire de la conduite de sang du côté du patient et un deuxième raccordement (112) du côté du patient pour un deuxième segment tubulaire de la conduite de sang du côté du patient,
un deuxième corps de clapet (102) qui présente un premier raccordement (106) du côté de la machine pour un premier segment tubulaire de la conduite de sang du côté de la machine et un deuxième raccordement (108) du côté de la machine pour un deuxième segment tubulaire de la conduite de sang du côté de la machine,
le premier et le deuxième corps de clapet (104, 102) étant formés de façon à pouvoir tourner autour d'un axe (144) de telle sorte que
dans une première position une liaison de fluide entre le premier raccordement (110) du côté du patient et le premier raccordement (106) du côté de la machine d'une part, et entre le deuxième raccordement (112) du côté du patient et le deuxième raccordement (108) du côté de la machine d'autre part soit produite et
dans une deuxième position une liaison de fluide entre le premier raccordement (110) du côté du patient et le deuxième raccordement (108) du côté de la machine d'une part, et entre le deuxième raccordement (112) du côté du patient et le deuxième raccordement (106) du côté de la machine d'autre part soit produite,
**caractérisé en ce que**
le dispositif présente pour l'inversion du flux :
un système de communication (210) pour recevoir un champ de haute fréquence électromagnétique d'un système de lecture du dispositif de traitement extracorporel du sang et pour générer au moins un identifiant caractérisant la position du corps de clapet qui peut être lu par le système de lecture,
le système de communication (210) présentant un premier transpondeur RFID (211) et un deuxième transpondeur RFID (212), le premier transpondeur RFID envoyant un identifiant caractérisant la première position et le deuxième transpondeur RFID envoyant un identifiant caractérisant la deuxième position.

2. Dispositif selon la revendication 1, **caractérisé en ce que** le premier transpondeur RFID (211) est disposé sur un emplacement caractérisant la première position sur le premier ou deuxième corps de clapet et le deuxième transpondeur RFID (212) est disposé sur un emplacement caractérisant la deuxième position sur le premier ou deuxième corps de clapet (104, 102),
et **en ce que** le premier et/ou le deuxième corps de clapet (104, 102) est protégé électriquement de telle sorte que dans la première position seul le premier transpondeur RFID (211) peut être lu pour envoyer l'identifiant caractérisant la première position par le système de lecture, et dans la deuxième position seul le deuxième transpondeur RFID (212) peut être lu pour envoyer l'identifiant caractérisant la deuxième position par le système de lecture.

3. Dispositif selon la revendication 2, **caractérisé en ce que** les premier et deuxième transpondeurs RFID (211, 212) sur la face interne du premier corps de clapet (104) sont disposés face à face sur un axe perpendiculaire à l'axe de rotation du corps de clapet, la face interne du deuxième corps de clapet étant métallisée à l'exception d'une encoche (213) pour la protection du transpondeur RFID (211, 212) qui est opposée dans la première position au premier transpondeur RFID et dans la deuxième position, au deuxième transpondeur RFID (212), de telle sorte que dans la première position seul le premier transpondeur RFID puisse être lu pour envoyer l'identifiant caractérisant la première position par le système de lecture, et que dans la deuxième position seul le deuxième transpondeur RFID puisse être lu pour envoyer l'identifiant caractérisant la deuxième position, par le système de lecture.

4. Dispositif selon l'une des revendications 1 à 3, **caractérisé en ce que** la face interne du premier corps de clapet (104) est métallisée pour la protection électrique du transpondeur RFID (211, 212).

5. Dispositif selon la revendication 1, **caractérisé en ce que** dans la première position seul l'émetteur-récepteur du premier transpondeur RFID (211) est relié à une antenne (214), et dans la deuxième position seul l'émetteur-récepteur du deuxième transpondeur RFID (212) est relié à une antenne, de telle sorte que dans la première position seul le premier transpondeur RFID pour envoyer l'identifiant caractérisant la première position puisse être lu par le système de lecture, et dans la deuxième position seul le deuxième transpondeur RFID puisse être lu pour envoyer l'identifiant caractérisant la deuxième position par le système de lecture.

6. Dispositif selon la revendication 5, **caractérisé en ce que** les émetteurs-récepteurs des premier et deuxième transpondeurs RFID (211) sont placés dans le premier corps de clapet (104) et dans le deuxième corps de clapet (212) est placée une antenne commune (214) pour les émetteurs-récepteurs des premier et deuxième transpondeurs RFID, un système de contact (214A ; 211 E, 212E) pouvant être produit pour la production d' une liaison électrique entre le premier émetteur-récepteur et l'antenne dans la première position, et pour la production d'une liaison électrique entre le deuxième émetteur-récepteur et l'antenne dans la deuxième position.

7. Dispositif selon la revendication 6, **caractérisé en ce que** le système de contact présente une première liaison électrique (211C, 212C ; 140, 150) pour la production d'une liaison électrique durable entre les premier et deuxième émetteurs-récepteurs et l'antenne (214), le système de contact présentant sur le premier corps de clapet (104) un contact (211 E) lié au premier émetteur-récepteur et un contact (212E) lié au deuxième émetteur-récepteur, et présentant sur le deuxième corps de clapet (102) un curseur (214A), le curseur pouvant être mis en contact dans la première position avec le premier contact et dans la deuxième position avec le deuxième contact.

8. Dispositif selon l'une des revendications 1 à 7, **caractérisé en ce que** les premier et deuxième transpondeurs RFID (211, 212) sont des transpondeurs passifs.

9. Dispositif pour inverser le flux sanguin pour un dispositif de traitement extracorporel du sang qui présente une conduite de sang artériel partant d'un patient, conduisant à une unité de traitement du sang et une conduite de sang veineux partant de l'unité de traitement du sang, conduisant au patient,
le dispositif présentant pour l'inversion du flux :
un premier corps de clapet (104) qui présente un premier raccordement (110) du côté du patient pour un premier segment tubulaire de la conduite de sang du côté du patient et un deuxième raccordement (112) du côté du patient pour un deuxième segment tubulaire de la conduite de sang du côté du patient,
un deuxième corps de clapet (102) qui présente un premier raccordement (106) du côté de la machine pour un premier segment tubulaire de la conduite de sang du côté de la machine et un deuxième raccordement (108) du côté de la machine pour un deuxième segment tubulaire de la conduite de sang du côté de la machine,
le premier et le deuxième corps de clapet (104, 102) étant formés de façon à pouvoir tourner autour d'un axe (144) de telle sorte que
dans une première position une liaison de fluide entre le premier raccordement (110) du côté du patient et le premier raccordement (106) du côté de la machine d'une part, et entre le deuxième raccordement (112) du côté du patient et le deuxième raccordement (108) du côté de la machine d'autre part soit produite et
dans une deuxième position une liaison de fluide entre le premier raccordement (110) du côté du patient et le deuxième raccordement (108) du côté de la machine d'une part, et entre le deuxième raccordement (112) du côté du patient et le premier raccordement (106) du côté de la machine d'autre part soit produite,
**caractérisé en ce que**
le dispositif présente pour l'inversion du flux :
un système de communication (210) pour recevoir un champ de haute fréquence électromagnétique d'un système de lecture du dispositif de traitement extracorporel du sang et pour générer au moins un identifiant caractérisant la position du corps de clapet qui peut être lu par le système de lecture,
le système de communication (210) présentant un transpondeur RFID (211) qui envoie un identifiant caractérisant la première position et un identifiant caractérisant la deuxième position.

10. Dispositif selon la revendication 9, **caractérisé en ce qu'**un système (216) détectant la première et la deuxième position du corps de soupape (104, 102) est prévu, qui coopère avec le transpondeur RFID (211) de telle sorte que l'identifiant caractérisant la première position soit lisible par le système de lecture dans la première position, et que l'identifiant caractérisant la deuxième position soit lisible par le système de lecture dans la deuxième position.

11. Dispositif selon la revendication 9 ou 10, **caractérisé en ce que** le transpondeur RFID (211) est un transpondeur passif.

12. Dispositif de traitement du sang pour réaliser un traitement extracorporel du sang, dans lequel dans une circulation sanguine extracorporelle (I) le sang d'un patient s'écoule par une conduite de sang artériel (108A) jusqu'à une unité de traitement de sang (204) et s'écoule de l'unité de traitement du sang par une conduite de sang veineux (106A) jusqu'au patient, dans les conduites de sang (106A, 108A) un dispositif (100) pour l'inversion du flux sanguin dans la circulation sanguine extracorporelle (1) étant activé selon l'une des revendications 1 à 11,
**caractérisé en ce que** le dispositif de traitement sanguin (100) présente un système de lecture (270) qui est conçu de telle sorte que l'identifiant caractérisant la première ou la deuxième position, du système de communication (210) soit lisible.

13. Dispositif de traitement du sang selon la revendication 12, **caractérisé en ce que** le dispositif de traitement du sang présente un système (240) de détermination d'un paramètre de traitement du sang sur la base d'une première mesure d'une grandeur caractéristique avant, et d'une deuxième mesure d'une grandeur caractéristique après l'inversion de l'écoulement sanguin,
le dispositif de traitement présentant une unité d'alarme (250) qui coopère avec le système (240) de détermination d'un paramètre de traitement du sang de telle sorte que l'unité d'alarme (250) émette une alarme lorsque après la première mesure le système de lecture (270) n'a pas reçu l'identifiant caractérisant la deuxième position, et lorsque après la deuxième mesure le système de lecture n'a pas reçu l'identifiant caractérisant la première position.

14. Dispositif de traitement du sang selon la revendication 12 ou 13, **caractérisé en ce que** le dispositif de traitement du sang présente un système (220) de reconnaissance du système de communication du dispositif correspondant pour l'inversion de l'écoulement sanguin, le système (220) de reconnaissance du dispositif de communication présentant des moyens qui sont conçus de telle sorte qu'un échange entre l'identifiant caractérisant la première et la deuxième position dans un intervalle temporel prédéterminé soit reconnu.

15. Procédé de détermination de l'inversion de l'écoulement du sang dans un traitement extracorporel du sang avec un dispositif de traitement extracorporel du sang qui présente un dispositif d'inversion de l'écoulement sanguin dans la circulation sanguine extracorporelle,
**caractérisé en ce qu'**un champ de haute fréquence électromagnétique est émis par un système de lecture (270) du dispositif de traitement du sang et est reçu par un système de communication (210) du dispositif pour l'inversion de l'écoulement, le système de communication (210) générant un premier identifiant caractérisant la direction d'écoulement dans la circulation sanguine extracorporelle dans une direction et un deuxième identifiant caractérisant la direction d'écoulement dans la circulation sanguine extracorporelle dans l'autre direction qui est lu par le système de lecture du dispositif de traitement du sang, et le premier identifiant d'un premier transpondeur RFID (211) et le deuxième identifiant d'un deuxième transpondeur RFID (212) étant émis ou les premier et deuxième identifiants d'un transpondeur RFID unique (211) étant émis.
